# EUROPÄISCHE PATENTANMELDUNG

(11) **EP 0 615 973 A1**
(43) Veröffentlichungstag der Anmeldung: **21.09.1994**
(21) Anmeldenummer: 94103871.3
(22) Anmeldetag: 14.03.1994
(51) Int. Cl.: C07H 15/04

(54) **Verfahren zur Herstellung von Alkylglycosiden**

(30) Priorität: 19.03.1993 EP 93104598
(71) Anmelder: Akzo Nobel N.V., NL-6824 BM Arnhem (NL)
(72) Erfinder: Bergfeld, Manfred Josef, Dr., D-63906 Erlenbach (DE); Seifert, Jürgen, Dr., D-63868 Grosswallstadt (DE)
(74) Vertreter: Fett, Günter

(57) **Zusammenfassung**

Es wird ein Verfahren zur Herstellung von Alkylglycosiden durch Umsetzung von Monosacchariden mit Fettalkoholen in Gegenwart eines sauren Katalysators beschrieben, das dadurch gekennzeichnet ist, daß man als Katalysator Sulfocarbonsäuren und deren Ester mit Ausnahme von Sulfobernsteinsäure und deren Dialkylester, deren Gesamtzahl der Kohlenstoffatome in den beiden Alkylgruppen mindestens 10 beträgt, sowie Anhydride von mehrwertigen Sulfocarbonsäuren verwendet.

## Beschreibung

Die Erfindung betrifft ein Verfahren zur Herstellung von Alkylglycosiden insbesondere durch direkte Synthese, ausgehend von Fettalkoholen und Sacchariden unter Verwendung von sauren Katalysatoren.

Unter Alkylglycosiden sind im Rahmen der Erfindung Verbindungen zu verstehen, bei denen Alkylreste acetalisch an monomere und/oder oligomere Zuckerreste gebunden sind.

Unter Alkylreste werden Reste verstanden, die sich von monofunktionellen Alkoholen ableiten, die linear oder verzweigt sein können und bevorzugt 8 - 20 Kohlenstoffatome besitzen. Besonders geeignete Alkylreste sind solche, die sich von Alkoholen ableiten, die aus Naturstoffen wie Fetten und Ölen gewonnen werden und meistens ein Gemisch darstellen, zum Beispiel C₁₂/C₁₄-Ketten besitzen und auch ungesättigte Reste aufweisen können.

Es ist bekannt, Alkylglycoside aus Fettalkoholen und Sacchariden unter Verwendung eines sauren Katalysators herzustellen. Es werden in der Literatur zahlreiche Methoden beschrieben, bei denen zunächst ein Glycosid aus Sacchariden durch Umsetzung eines niedermolekularen Alkohols wie Methanol, Äthanol u.dgl. gewonnen wird, das sodann durch Umacetalisierung mit einem höhermolekularen Alkohol in das gewünschte Alkylglycosid umgewandelt wird. Für diesen Reaktionstyp sind zahlreiche Katalysatoren empfohlen worden.

Es ist ferner bekannt, aus Sacchariden direkt durch Umsetzung mit einem höhermolekularen Alkohol, d.h. Alkohole mit 8 bis beispielsweise 22 Kohlenstoffatomen zu synthetisieren. Auch für diese Reaktionsweise sind in der Literatur bereits zahlreiche saure Katalysatoren erwähnt.

So werden, wie z.B. der europäischen Patentanmeldung 0 132 043 zu entnehmen ist, anorganische Säuren wie Salzsäure, Schwefelsäure, Phosphorsäure empfohlen; auch p-Toluolsulfonsäure, Bortrifluorid werden zu diesem Zweck angegeben. Nach der Lehre der europäischen Patentanmeldung 0 132 043 werden saure anionische, oberflächenaktive Mittel wie Alkylsulfate, Alkylbenzolsulfonsäuren und Alkylsulfonsäuren als Katalysatoren für die Herstellung von Alkylglycosiden eingesetzt.

In der europäischen Patentanmeldung Nr. 0 415 192 wird als Katalysator Sulphobernsteinsäure empfohlen, weil diese biologisch leicht abbaubar ist.

In der internationalen Patentanmeldung WO 90/07516 werden für die Herstellung von Alkylglycosiden als Katalysatoren ganz allgemein starke hydrophobe organische Säuren empfohlen. Hierbei handelt es sich vor allem um Verbindungen, die oberflächenaktiv sind. Neben Alkylbenzolsulfonsäuren, Schwefelsäurealkylestern, Copolymeren von Styrolsulfonsäuren werden unter anderem Dialkylester von Sulphobernsteinsäure mit insgesamt mindestens 10 Kohlenstoffatomen in den Alkylgruppen ganz allgemein erwähnt. Beispiele für diese Alkylester sind dieser Patenanmeldung nicht zu entnehmen.

Obwohl bereits zahlreiche Katalysatoren für die indirekte und direkte Synthese von Alkylglycosiden benannt worden sind, besteht noch ein Bedürfnis nach verbesserten Verfahren der Direkt-Synthese, die unter Verwendung von sauren Katalysatoren arbeiten.

Aufgabe der Erfindung ist es deshalb ein Verfahren insbesondere zur Direkt-Synthese von Alkylglycosiden ausgehend von Fettalkoholen mit 8 - 22 Kohlenstoffatomen und Sacchariden, insbesondere Monosacchariden unter Verwendung von sauren Katalysatoren zur Verfügung zu stellen, das wirtschaftlich durchzuführen ist, das für gegebene Reaktionsbedingungen kurze Reaktionszeiten ermöglicht, das zu einem Produkt mit guten Eigenschaften führt und das einfach handhabbar ist und zu reproduzierbaren Ergebnissen führt. Aufgabe der Erfindung ist es ferner, ein Verfahren zur Verfügung zu stellen, das kontinuierlich durchgeführt werden kann und das zu Produkten führt, die günstige Viskositätseigenschaften aufweisen, eine zufriedenstellende Solubilisierung besitzen und für die Praxis zufriedenstellende Grenzflächeneigenschaften aufweisen.

Diese Aufgabe wird gelöst durch ein Verfahren zur Herstellung von Alkylglycosiden durch Umsetzung von Monosacchariden mit Fettalkoholen in Gegenwart eines sauren Katalysators, das dadurch gekennzeichnet ist, das man als Katalysator Sulfocarbonsäuren mit Ausnahme von Sulfobernsteinsäure und/oder Sulphocarbonsäureester mit Ausnahme von Sulfobernsteinsäure-dialkylestern, deren Gesamtzahl der Kohlenstoffatome in den beiden Alkylgruppen mindestens 10 beträgt, und/oder Anhydride von mehrwertigen Sulfocarbonsäuren verwendet.

Die erfindungsgemäß verwendeten Sulfocarbonsäuren bzw. deren Ester können aliphatischer, cycloaliphatischer, aromatischer oder heterocyclischer Natur sein. Sie können übliche Substituenten tragen. Es können Sulfomono-, Sulfodi-, Sulfotri- und mehrwertige Carbonsäuren bzw. deren Ester sein. Von den mehrwertigen Carbonsäuren, d.h. Sulphodicarbonsäuren, Sulphotricarbonsäuren u.s.w. können Mono-, Di-, Tri- u.s.w. -ester eingesetzt werden.

Bei den Alkoholen, von denen sich die Ester ableiten, handelt es sich um Alkohole mit im allgemeinen 1 - 22 Kohlenstoffatomen, wobei der Alkohol sich von aliphatischen, olefinischen, cycloaliphatischen und araliphatischen Resten ableitet.

In einer besonders vorteilhaften Ausführungsform des erfindungsgemäßen Verfahrens leiten sich die Ester von denselben Fettalkoholen ab, die als Reaktand bei der Umsetzung eingesetzt werden.

Im folgenden werden Beispiele von Säuren und Estern aufgeführt, die sich erfindungsgemäß als vorteilhaft erwiesen haben: Sulfoessigsäure und deren Methylester, Sulfoessigsäuredodecylester, Ortho-, Meta-, Parasulfobenzoesäure und deren Alkylester wie Methyl-, Dodecyl-, Tetradecylester, Sulfobernsteinsäuremonododecylester, Sulfobernsteinsäuredimethylester, Sulfopropionsäure und deren Dodecylester, Sulfoisophthalsäure und deren Mono- oder Didodecylester,
Sulfophthalsäureanhydrid,
5-Sulphotrimelithsäure und Mono-, Di- und Tridodecylester, 4-Sulpho-1,8-naphthalindicarbonsäuredodecylester, 4-Sulfo-1,8-naphthalindicarbonsäureanhydrid.

Bei Sulfocarbonsäuren mit zwei oder mehreren Carboxylgruppen können vorteilhaft auch deren Anhydride verwendet werden.

Anstelle der vorstehend genannten Dodecylester wurden auch Gemische, zum Beispiel von Dodecyl/Tetradecylestern oder Hexadecyl-/Octadecylestern verwendet, die sich von Naturprodukten ableiten.

Die Menge des Katalysator kann in verhältnismäßig weiten Bereichen variiert werden wie z.B. 1 bis 20 mmol pro mol Glucose. Bevorzugt werden 2 bis 10 mmol pro Mol Glucose.

Es können vorteilhaft auch Gemische der oben definierten Säuren miteinander bzw. Gemische der Säuren mit vorstehend benannten Estern oder schließlich Gemische der Ester an sich eingesetzt werden. Das gleiche gilt analog auch für die Anhydride.

Als Monosaccharid wird vorzugsweise Glucose verwendet, besonders geeignet ist eine feingemahlene Glucose mit einem mittleren Teilchendurchmesser von 3 - 4 µm.

In einer besonders vorteilhaften Ausführungsform des erfindungsgemäßen Verfahrens wird die Umsetzung kontinuierlich durchgeführt.

Direkte Synthese im Rahmen der Erfindung bedeutet, daß die Reaktanden Fettalkohol und Monocaccharid direkt zum Alkylgykosid umgesetzt werden ohne den Zwischenschritt der Umacetalisierung eines Zwischenprodukts mit einem kürzerkettigen Alkohol.

Es war besonders überraschend, daß die Erfindung es ermöglicht, ein Verfahren zur Verfügung zu stellen, bei dem eine große Zahl von neuen Katalysatoren für diesen Zweck eingesetzt werden können. Das Verfahren arbeitet sehr wirtschaftlich, ist gut handhabbar und liefert reproduzierbar Produkte mit einstellbaren Eigenschaften.

Es war ferner besonders überraschend, daß sich das erfindungsgemäße Verfahren auch auf einfache Weise kontinuierlich durchführen läßt und zu Produkten führt, die verglichen mit Produkten, die im Handel sind, bei sonst gleicher Performance erheblich niedrigere Viskositäten aufweisen. Dies bedeutet, daß es möglich ist auch noch bei höheren Konzentrationen wäßrige Systeme zur Verfügung zu stellen, die bei Zimmertemperatur pumpbar sind. Grenzflächenspannungswerte und die Solubilisierung der Produkte sind hervorragend; auch die Schaumstabilität ist gut.

Es ist ferner überraschend, daß die Katalysatoren, insbesondere bei kontinuierlicher Arbeitsweise zu sehr hohen Reaktionsgeschwindigkeiten führen. Es ist ferner überraschend, daß gemäß der Erfindung die Reaktion sich vielfach bei niedrigeren Temperaturen durchführen läßt und trotzdem zu hohen Ausbeuten führt. Ein weiterer Vorteil der Erfindung ist darin zu suchen, daß man sirupöse Glucose umsetzen kann, wie man sie zum Beispiel durch saure oder enzymatische Hydrolyse von Stärke erhält. Diese sirupösen Glucosen enthalten neben Glucose, also einem Monosaccharid und Wasser auch noch Oligomere und zu einem geringeren Teil Polysaccharide. Sirupöse Glucose ist auch erhältlich aus Saccharose die man beispielsweise aus Zuckerrüben gewinnt.

Die Erfindung wird anhand folgenden Beispiels näher erläutert:

### Allgemeine Vorgehensweise und Beschreibung der verwendeten Apparatur

Als Reaktor wurde ein handelsüblicher 1 L Büchi-Glasreaktor mit Doppelmantelbeheizung, Intermig-Rührer, Bodenablaß und Destillationsaufsatz verwendet.

Die folgenden Beispiele wurden im batch-Betrieb gefahren, d.h. die gesamte Glucose (Glc) und der gesamte Fettalkohol (FA) wurden im Reaktor vorgelegt und das Gemisch unter Rühren und Evakuieren auf 20 mbar auf Solltemperatur gebracht.

Danach wurde der Katalysator zugegeben und dieser Zeitpunkt als Reaktionsbeginn gewertet.

Das Reaktionswasser wurde kontinuierlich als Dampf abgezogen und in ein graduiertes Gefäß niedergeschlagen. Die Wasserentstehungsrate wurde als Stoffänderungsgeschwindigkeit der Glc gewertet (pro Mol umgesetztes Glc wird 1 Mol Wasser frei).

Die notwendige Zeit für 99% Glc-Umsatz wurde als Reaktionszeit gewertet und für die angestellten Vergleiche verwendet.

### Beispiel

Als Katalysator wurden 10 mmol 4-Sulfophthalsäuredilaurylester pro mol Glucose verwendet.

90.5 g Glucose wasserfrei (Cerestar) mit einem mittleren Korndurchmesser von 5 µm wurden zusammen mit 412.0 g Fettalkohol (Nafol 1214 von Condea enthaltend ca. 54% Lauryl- und ca. 44% Myristylalkohol) im Reaktor vorgelegt und nach Evakuieren auf 20 mbar unter Rühren auf die Reaktionstemperatur von 110°C gebracht.

Nach 32 min waren 99% der vorgelegten Glucose umgesetzt. Das Reaktionsprodukt war klar, weiß und zeigte nach Abtrennung des FA auf 1.2% folgende Analysenwerte:

| | |
|---|---|
| freie Glucose: | < 1 Gew.% |
| Monoglucosid C₁₂ + C₁₄): | 53,5 Gew.% |

### Vergleichsbeispiel

Dieses Beispiel wurde analog mit einem Katalysator nach dem Stand der Technik (p-Toluolsulfonsäure) gefahren.
Für 99% Glucose-Umsatz waren hierbei jedoch 85 min, also mehr als die doppelte Reaktionszeit im Vergleich zu einem erfindungsgemäßen Katalysator erforderlich.

Das Reaktionsprodukt war klar und von hellgelber Farbe; es wies nach einer. FA-Abtrennung auf 1.6% Restgehalt folgende Analysenwerte auf:

| | |
|---|---|
| freie Glucose: | < 1 Gew.% |
| Monoglucosid C₁₂ + C ₁₄): | 52,4 Gew.% |

## Patentansprüche

1. Verfahren zur Herstellung von Alkylglycosiden durch Umsetzung von Monosacchariden mit Fettalkoholen in Gegenwart eines sauren Katalysators, dadurch gekennzeichnet, daß man als Katalysator Sulfocarbonsäuren mit Ausnahme von Sulfobernsteinsäure und/oder Sulphocarbonsäureester mit Ausnahme von Sulphobernsteinsäuredialkylestern, deren Gesamtzahl der Kohlenstoffatome in den beiden Alkylgruppen mindestens 10 beträgt, und/oder Anhydride von mehrwertigen Sulfocarbonsäuren verwendet.

2. Verfahren nach Anspruch 1, dadurch gekennzeichnet, daß man Ester von aliphatischen und/oder cycloaliphatischen Sulphocarbonsäuren verwendet.

3. Verfahren nach Anspruch 1, dadurch gekennzeichnet, daß man Ester von aromatischen Sulphocarbonsäuren verwendet.

4. Verfahren nach einem der Ansprüche 1 bis 3, dadurch gekennzeichnet, daß man Ester verwendet, die sich von aliphatischen, olefinischen, cycloaliphatischen und araliphatischen Alkoholen mit bis zu 22 Kohlenstoffatomen ableiten.

5. Verfahren nach einem oder mehreren der Ansprüche 1 bis 4, dadurch gekennzeichnet, daß man Sulphocarbonsäureester verwendet, deren Alkoholkomponente die gleiche ist, wie der zur Umsetzung als Reaktand verwendete Fettalkohol.

6. Verfahren nach einem oder mehreren der Ansprüche 1 bis 5, dadurch gekennzeichnet, daß man als Monosaccharid Glucose verwendet.

7. Verfahren nach Anspruch 6, dadurch gekennzeichnet, daß man eine feingemahlene Glucose verwendet.

8. Verfahren nach Anspruch 7, dadurch gekennzeichnet, daß man eine Glucose mit einem Teilchendurchmesser von 3 bis 4 µm verwendet.

9. Verfahren nach einem oder mehreren der Ansprüche 1 bis 8, dadurch gekennzeichnet, daß man die Umsetzung kontinuierlich durchführt.
